# EUROPÄISCHE PATENTSCHRIFT

(11) **EP 3 366 931 B1**
(45) Veröffentlichungstag und Bekanntmachung des Hinweises auf die Patenterteilung: **16.06.2021**
(21) Anmeldenummer: 17157462.7
(22) Anmeldetag: 22.02.2017
(51) Int. Cl.: F16B 13/12

(54) **TÜLLE UMFASSEND EINE SENSORANORDNUND MIT RFID**
SLEEVE COMPRISING A SENSOR ARRANGEMENT WITH RFID
DOUILLE COMPORTANT UN ARRANGEMENT DE SENSOR AVEC RFID

(43) Veröffentlichungstag der Anmeldung: 29.08.2018
(73) Patentinhaber: SFS Intec Holding AG, 9435 Heerbrugg (CH)
(72) Erfinder: Gasser, Daniel, 9444 Diepoldsau (CH); Blum, Kurt, 6842 Koblach (AT)

(56) Entgegenhaltungen:
- EP-A1- 1 175 567
- JP-A- 2015 215 226
- KR-A- 20070 092 374
- US-A1- 2010 050 778

## Beschreibung

Die vorliegende Erfindung befasst sich mit einer Sensoranordnung in Gebäudehüllen, insbesondere zur Messung von Umweltparametern und Erkennung von Bauschäden.

### HINTERGRUND

Im Bauwesen sind im zunehmenden Masse hochkomplexe Gebäudehüllen im Einsatz, die aus mehrlagigen, funktionell abgestimmten Schichten bestehen. Dabei werden sowohl bei Aussenwand wie auch Dachkonstruktionen Kombinationen von thermischen Isolationsschichten, tragenden/fixierenden Elementen und Schutzmembranen gegen Umwelteinflüsse wie Wind, Regen, Schnee etc. eingesetzt. Kombinationen von Mineralfaserprodukten, Schaumstoffen, Kunststofffolien und punktförmigen Befestigungselementen dafür sind speziell bei Dachaufbauten heute üblich.
Solche Gebäudehüllen dauerhaft dampf- und wasserdicht zu halten erfordert nicht nur eine abgestimmte Auswahl von Produkten, sondern auch hohe Sorgfalt bei der Verarbeitung und Montage. Bei Industriedächern mit teilweise Tausenden von Quadratmetern, wo Hunderte von Metern an Schweissnähten zwischen Kunststofffolienbahnen anfallen können, sind die Anforderungen besonders hoch. Erschwerend kommt hinzu, dass die zunehmend dicken Isolationslagen je nach Material Wasser aufnehmen können, was dazu führt, dass eventuelle Leckagen erst spät entdeckt werden können und dann der entstandene Schaden bereits hoch ist.

### STAND DER TECHNIK

Es sind im Stand der Technik verschiedene Lösungen für dieses Problem beschrieben. Zum einen werden Detektionsverfahren für Leckagen eingesetzt, die auf verschiedenen Diagnosemethoden beruhen. Es gibt elektrische Verfahren, bei denen über ein Geflecht von in der Unterkonstruktion angebrachten Leitungen Leckströme messbar werden, sobald sich durch einen lokalen Wassereinbruch die elektrische Isolationswirkung des Dachaufbaus verändert. Alternativ können Dichtheitsmessungen mit Überdruck erfolgen oder aber über bildgebende Thermografie, wenn durch die Leckage eine Wärmebrücke entsteht. Allen Verfahren gemein ist ein hoher Aufwand bei der Anwendung bzw. bei der Installation.

Es wurden auch bereits Verfahren beschrieben, die mit Hilfe von RFID-gekoppelten Sensoren arbeiten. RFID Systeme (radio-frequency identification) verwenden ein als Transponder bezeichnetes Halbleiterbauelement mit einer Antenne und ein dazu passendes Lesegerät. Solche RFID-Systeme haben den Vorteil, dass über die Antenne nicht nur die Identifikation des Transponders erfolgt, sondern auch die Energieversorgung via magnetische Wechselfelder oder durch hochfrequente Radioimpulse (Induktion). Diese Energieversorgung reicht auch aus, um einfache Sensoren zu betreiben, z.B. Feuchtesensoren oder Temperaturfühler. Kombiniert mit nicht-flüchtigen Speichern lassen sich so Messnetze aufbauen, die ohne Verkabelung und eigene Stromversorgung auskommen können.

So zeigt die Schrift JP 2015-215226 A eine Zustands-Überwachungsvorrichtung (state detection device), die dafür ausgelegt ist, einen Zustand zu überwachen. Konkret wird eine, in eine äussere Struktur einschraubbare, schraubenähnliche Hülse vorgeschlagen, die in ihrem Inneren eine Sensoranordnung aufweist, wobei die Kommunikationsantenne im Kopf der Vorrichtung untergebracht wird. Figur 9 und die zugehörige Offenbarung beschreiben, dass der eigentliche Sensor (probe electrode 11a) in der Spitze untergebracht ist und auch aus einer Öffnung dort vorstehen darf und mit der umliegenden Struktur in Kontakt treten kann.

Die koreanische Offenlegungsschrift KR 20070092374 A beschreibt eine als Nagel geformte Vorrichtung (Geo-Informationspunkt) mit einer elektronischen Plakette, die in einen Untergrund eingebracht_werden kann. Sie wird drahtlos ausgelesen und erlaubt so den Zugriff auf eine Datenbank mit Informationen über die Umgebung, z.B. Leitungen im Untergrund. Es wird vorgeschlagen RFID-Technologie zu nutzen.
Nachteilig bei bekannten Lösungen ist, dass diese Sensoren mit RFID Transponder separat verlegt werden müssen, z.B. unterhalb oder zwischen verschiedenen Schichten der Gebäudehülle. Auch eine definierte Einbaulage bzw. Einbautiefe (relativ z.B. zur Oberfläche einer Dachfläche) muss gewährleistet sein - einerseits um eine Vergleichbarkeit der Messergebnisse zu erlauben aber auch wegen der Erreichbarkeit des Sensors selbst.

Das Dokument US 2010/0050778A1 beschreibt einen Befestiger mit einer Sensoranordnung und einem Transponder, der in der Lage ist, den Zustand der Verbindung (durch Messung der Zuglast auf den Befestiger) festzustellen und die Werte elektronisch einer Überwachungsstation zu übermitteln. Der Sender ist dazu im Kopf des Befestigers untergebracht.

Die vorliegende Erfindung befasst sich mit dem oben beschriebenen Problem und schlägt, basierend auf den Merkmalen des Hauptanspruchs 1, eine erfinderische Lösung vor. Die Unteransprüche beschreiben weitere Varianten und Ausbaustufen der Erfindung.

### BESCHREIBUNG DER ERFINDUNG

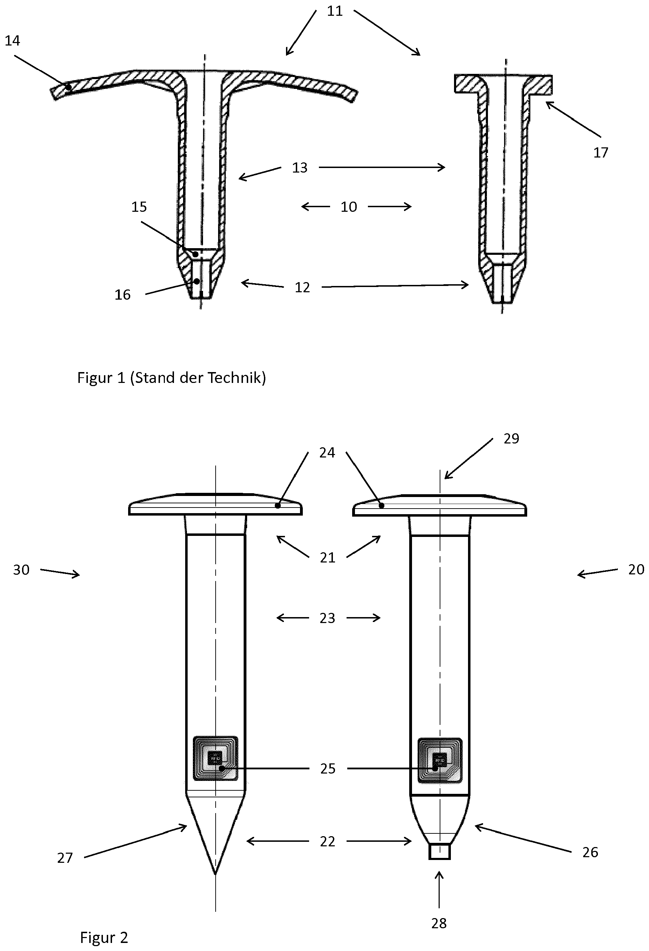

In der Gebäudetechnik sind seit langem Befestigungselemente bekannt, bei denen eine Kombination aus einer Plastiktülle und einem Schraubelement verwendet wird, um eine Dachisolationsbahn auf einem festen Unterbau zu befestigen. Die EP 1 175 567 A (ähnlich Fig. 1) zeigt eine Tülle 10, die im Wesentlichen aus einem Kopf 11 mit einer grossflächigen Unterlagscheibe 14 besteht, an deren Mittenloch eine rohrförmige Hülse 13 anschliesst, die sich am kopffernen Ende konusförmig auf einen kleineren Durchmesser verengt. Die Hülse kann eine Schraube aufnehmen (in Figur 1 nicht gezeigt), deren Schaft den Konus durch eine axiale Öffnung 16 passieren kann, deren Kopf jedoch an der Konus-Verengung 15 anschlägt. Durch Wahl verschieden langer Hülsen bzw. Tüllen können mit einem Schraubentyp bzw. -länge verschieden dicke Dachisolationsbahnen fixiert werden.

Bekanntermassen können diese Tüllen unterschiedlich ausgeführt werden, sowohl was die Länge der Hülse 13 angeht, wie auch Grösse und Material der grossflächigen Unterlagscheibe 14. So ist bekannt, die Hülse 13 und die Unterlagscheibe 14 getrennt zu fertigen. Somit können Hülsen 13 verschiedener Länge und Unterlagscheiben 14 verschiedenen Durchmessers und Form (eckig, rund, oval,...) kombiniert werden, was die Einsatzmöglichkeiten erhöht. Zudem ist es bekannt die Unterlagscheiben 14 z.B. aus geprägtem / gestanztem Stahlblech oder aus Kunststoff zu fertigen. Dann wird in der Regel die Hülse 13 so ausgelegt, dass sie an ihrem der Spitze abgewandtem Ende einen Bund aufweist, einen umlaufenden, radial vorstehenden Rand, der für eine von der Spitze her aufgeschobene Unterlagscheibe 14 als Anschlag wirkt. Dazu können Elemente kommen, die diese Unterlagscheibe lösbar oder dauerhaft fixiert an ihrem Platz halten, wie Rastelemente, Klemmen, Klebungen, etc. Wenn im Folgenden von "Tülle" die Rede ist, ist die grundsätzlichste Ausführungsform gemeint. "Tülle mit grossflächiger Unterlagscheibe" umfasst daher sowohl die ein- wie auch die zweistückige Ausführung.

Die Erfindung schlägt nun vor, ein Befestigungselement der genannten Art mit einem RFID Transponder und einer Sensorik zu versehen. Dadurch kann - ohne einen zusätzlichen Bearbeitungsschritt vorzusehen, in einer definierten Einbaulage und -tiefe ein Sensor in einer Schicht eines Dachaufbaus eingebracht werden. Ebenso kann eine zusätzliche Verletzung der Isolationsschichten bzw. der Dachmembran vermieden werden - es wird der ohnehin vorhandene Befestigungspunkt verwendet. Ferner kommt erleichternd hinzu, dass in der Branche das Knowhow zur Verlegung solcher Tülle/Schraube-Systeme vorhanden ist, eine Umschulung oder Umstellung auf das neue System kann denkbar einfach erfolgen. Zudem kann die Planung vereinfacht werden. Bereits jetzt werden solche Befestigungselemente an (z.B. durch Wind oder Wechselbelastung) hochbeanspruchten Dachbereichen in höherer Anzahl pro Quadratmeter verbaut. Die Anzahl von Messstellen an solchen Flächen zu erhöhen (sei es aus Gründen der Redundanz oder Sicherheit) lässt sich so ohne grossen Planungsaufwand realisieren.

Dort, wo ein Befestigungspunkt mit Sensorelement nicht notwendig erscheint, können Befestigungselemente (Tülle/Schraube) gemäss Stand der Technik verwendet werden, ohne den Setzvorgang selbst zu verändern. Durch unterschiedliche Farbkennzeichnung können die "intelligenten" RFID-Setzpunkte von den normalen Befestigungspunkten unterschieden werden. Bei Verwendung von Magazinstreifen, in denen die Befestigungselemente gebündelt in ein Setzgerät eingelegt werden, lässt sich die Anzahl bzw. das Verhältnis von "intelligenten" zu normalen Befestigungselementen einstellen und so ein Irrtum bei der Montage von vornherein vermeiden.

Im Kern besteht eine Tülle 20, 30 im Wesentlichen aus einem Kopf 21, einer Spitze 22 und einer Hülse 23 dazwischen, wobei der Kopf 21 eine als (je nach Einsatzfall grossflächige, lösbare oder fixe) Unterlagscheibe 24 ausgebildet sein kann oder als Anschlagbund 17. Die äussere Form der Unterlagscheibe 24 kann (bevorzugt) kreisrund, weiterhin als Oval, oder eckig mit und ohne Kantenverrundung ausgeführt sein. An den Kopf 21 schliesst sich die rohrförmige Hülse 23 an, die wiederum in die Spitze 22 mündet. Diese Spitze kann bevorzugt im Wesentlichen eine Konus- (26) oder Kegelform 27 aufweisen und verengt sich daher auf einen kleineren Durchmesser als die Hülse 23 besitzt. Erfindungsgemäss wird die Tülle 20, 30 eine Sensoranordnung 25 aufweisen, mit zumindest einem RFID Transponder mit Antenne und einen mit dem Transponder wirkverbundenen Sensor.

Diese Tüllen sind, wie die im Stand der Technik Bekannten, als Spritzgiessteil aus Kunststoff herstellbar. Die Sensoranordnung kann dabei vor dem Spritzgiessvorgang in das Werkzeug eingelegt werden und so vom Kunststoff der Tülle ganz oder teilweise umschlossen werden. Alternativ ist auch ein nachträgliches Befestigen bzw. Anbringen der Sensoranordnung an der Tülle möglich. Wie im Stand der Technik können das Hülsenelement mit der Spitze und die Unterlagscheibe separat angefertigt und vor der Montage zusammengesteckt (lösbar oder z.B. durch Rast- oder Klemm- bzw. Klebeelemente unlösbar) verbunden werden.

Die Tülle 20, 30 zeichnet sich dadurch aus, dass die Sensoranordnung 25 flächig auf der Aussenseite der Hülse 23 an der Spitze 22 angebracht ist. Dadurch ist der Sensor wie auch die Antenne des Transponders nach aussen erkennbar und die Messung erfolgt im direkten Kontakt mit dem umgebenden Material.

Alternativ, aber nicht als Teil der beanspruchten Erfindung kann die Sensoranordnung auf der Tülle 20, 30 räumlich verteilt auf bzw. an der Tülle angeordnet werden. Räumlich verteilt meint hierbei eine bewusste räumliche Trennung des Messfühlers (Sensors) an sich vom Transponder mit Antenne. Dazu befindet sich der Transponder mit Antenne kopfnah an der Hülse 23 oder an der Unterlagscheibe 24 selbst, während der Sensor nahe der Spitze an der Hülse 23 oder an der Spitze 22 selbst angebracht ist. Dadurch können z.B. bei Temperaturmessungen die Messpunkte immer in einer definierten Tiefe der Isolationsschicht angebracht werden, während die Antenne selbst näher an der Oberfläche der Gebäudehülle liegt, was die Empfangs- und Sendebedingungen verbessert. Da die Tüllen beim Verlegen alle möglichst identische Einbaulagen einnehmen sollen, liegen auch alle Sensoren in derselben Tiefe, was die Vergleichbarkeit resultierender Messergebnisse über eine Dachfläche enorm erleichtert.

Je nach Auslegung kann es auch vorteilhaft sein, eine Tülle 20, 30mit einer lösbar angebrachten Sensoranordnung 25 vorzusehen. Hier liegt der Vorteil in der Flexibilität der Anordnung der Sensorik, falls gefordert.

Alternativ und eher bevorzugt ist die Sensoranordnung 25 unlösbar mit der Tülle verbunden. Die Sensoranordnung kann aufgeklebt, angeheftet, angeschweisst, vergossen oder als Teil des Herstellvorgangs zumindest teilweise in die Tülle integriert werden. Aufkleben oder Anheften bieten sich an, wenn die Sensoranordnungen als separates Dünnschichtbauteil auf einer Klebefolie vorliegen oder zum Aufschweissen vorgesehen sind. Denkbar ist ebenfalls, die Bauteile, die Antenne und/oder die Leiterbahnen in einem Druckverfahren, z.B. durch ein Transferdruckverfahren anzubringen. Das hätte vor allem bei nicht-planen Unterlagen deutliche Vorteile.

In einer erfindungsgemässen Variante wird eine Tülle 20 an bzw. in der Spitze 22 eine zur Zentralachse 29 der Hülse 23 koaxiale Durchtrittsöffnung 28 aufweisen. Diese dient zum Durchtritt eines Befestigers 41, der kopfseitig mit der Spitze voran in die Tülle eingeführt werden kann. Die Befestigung an einer Gebäudehülle erfolgt danach wie aus dem Stand der Technik bekannt. Dabei wird die Tülle 20, ergänzt um den Befestiger 41 zu einem Befestigungselement 40, mit dem Isolationsschichten 42 auf einem festen Unterbau 43 befestigt werden können. Je nach Einsatzprofil bzw. Auslegung kann der Befestiger 41 als Teil des Befestigungselements 40 eine Holzschraube, eine Blechschraube, eine Betonschraube, ein Bolzen oder ein Niet sein.

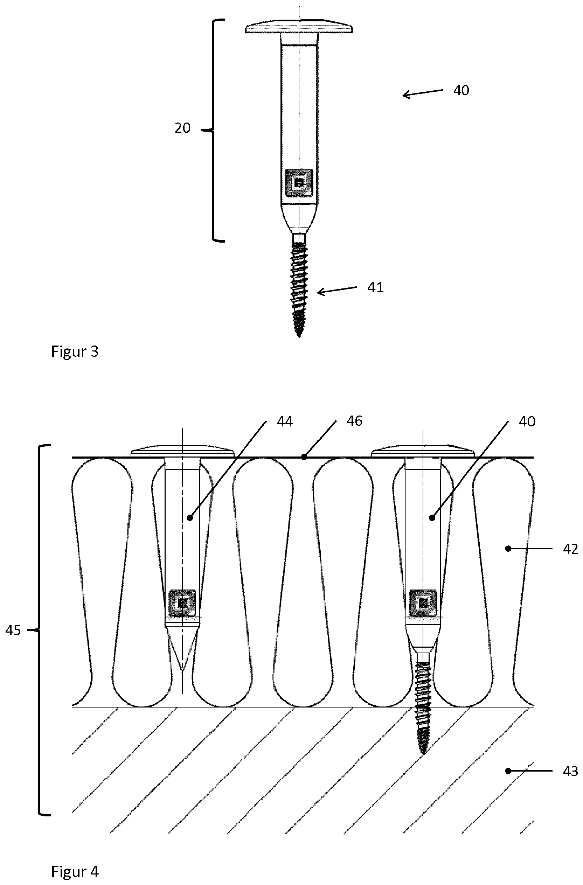

Im Sinne dieser Erfindung umfasst eine Tülle sowohl Varianten mit und ohne Durchtrittsöffnung 28. So wäre denkbar und vorteilhaft, eine Tülle rein als einsteckbares Element in eine Dachbahn bzw. Isolationsschicht einzusetzen, auch ohne sie mit einem Befestiger 41 als Befestigungselement 44 (siehe Figur 4) zu verwenden ("Einstecktülle"). Die Vorteile der tiefengenauen Anbringung bleiben erhalten, ebenso die gewohnte Montage zusammen mit anderen Befestigungselementen 40. So eine Tülle 44 kann eine Spitze aufweisen, wie in Figur 2 links gezeigt, die die Penetration des Isolationsmaterials 42 erleichtert.

In dem Zusammenhang sei auch verwiesen auf eine Sensoranordnung 25, umfassend einen RFID Transponder mit Antenne und mindestens einen mit dem Transponder wirkverbundenen Sensor. Die Sensoranordnung ist ausgelegt, als Teil eines Befestigungselements 40 eingesetzt zu werden, welches wiederum als Teil eines Befestigungssystems 45 für eine Gebäudehülle verwendbar ist.

Auch wenn die Beschreibung nicht alle möglichen Kombinationen der Merkmale direkt ausformuliert, ist damit die Kombinierbarkeit solcher Merkmale nicht ausgeschlossen.

### BESCHREIBUNG DER FIGUREN

Figur 1 zeigt ein Befestigungselement 10 nach dem Stand der Technik in zwei Varianten. In der Ausführung links ist der Kopf 11 ist als eine grossflächige Unterlagscheibe 14 ausgebildet, die, wie in Figur 4 gezeigt, auf einer Isolationsschicht einer Dachhülle aufliegen kann und dann die vertikalen Zugkräfte verteilt. Die Variante in Figur 1 rechts besitzt nur einen Anschlagbund 17, der mit verschiedenen Unterlagscheiben kombiniert werden kann. Eine rohrförmige Hülse 13 verbindet jeweils den Kopf 11 mit einer konischen Spitze 12, wie im Schnitt gezeigt. Die Hülse verengt sich am im Konus bei Merkmal 15 und geht über in eine axiale Öffnung 16.
Figur 2 zeigt die zwei bevorzugten Hauptvarianten der erfindungsgemässen Tülle. Die Variante in der Figur 2 rechts entspricht einer Tülle 20, deren Verwendungszweck die Funktion als Teil eines Befestigungselementes einschliesst, wie in Figur 4 illustriert ("Befestigungstülle"). Die Variante einer Tülle 30, im Bild links, entspricht dem reinen Einsteckelement ohne Befestigungsfunktion ("Einstecktülle"). Der Aufbau aus Kopf 21 mit Unterlagscheibe 24, Hülse 23 und Spitze 22 entspricht im Wesentlichen dem von Figur 1 und ist für Tülle 20 wie 30 vergleichbar. Die Form der Spitze 22 ist technisch bedingt und wird vom Fachmann unter Berücksichtigung fertigungstechnischer Vorgaben so ausgelegt werden, wie es der Einsatzzweck erfordert. Für die Einstecktülle 30 mit Spitze 27 empfiehlt sich eine geschlossene Spitze. Die Befestigungstülle 20 weist an der Spitze die Durchtrittsöffnung 28 auf, um einen Befestiger 41 aufzunehmen wie in Figur 3 bzw. Figur 4 gezeigt. Diese Öffnung ist koaxial mit der Zentralachse 29 der Hülse 23. Die Anbringung der Sensoranordnung 25 ist exemplarisch gezeigt, illustriert aber, dass sowohl die Befestigungstülle wie auch die Einstecktülle dieselbe Einbaulage und -tiefe erlauben.
Figur 3 zeigt die Kombination einer Befestigungstülle 20 mit einem eingesteckten Befestiger 41 als Befestigungselement 40.
Figur 4 zeigt ein Befestigungssystem 45 mit einem festen Unterbau 43, einer darauf angeordneten Isolationsschicht 42 und einer Deckfolie 46. Der Befestiger 41 eines Befestigungselements 40 ist in dem Unterbau 43 verankert, während die Einstecktülle 48 ohne Befestiger auskommt. Die Einbautiefe der Sensoranordnung ist dieselbe. Dies ist kein zwingendes merkmal, durch die unterschiedliche Anordnung gleicher Sensortypen liessen sich ohne weiteres Querschnittsmessungen eines gewählten Parameters (Temperatur, Feuchte,...) über die Schicht 42 erreichen.

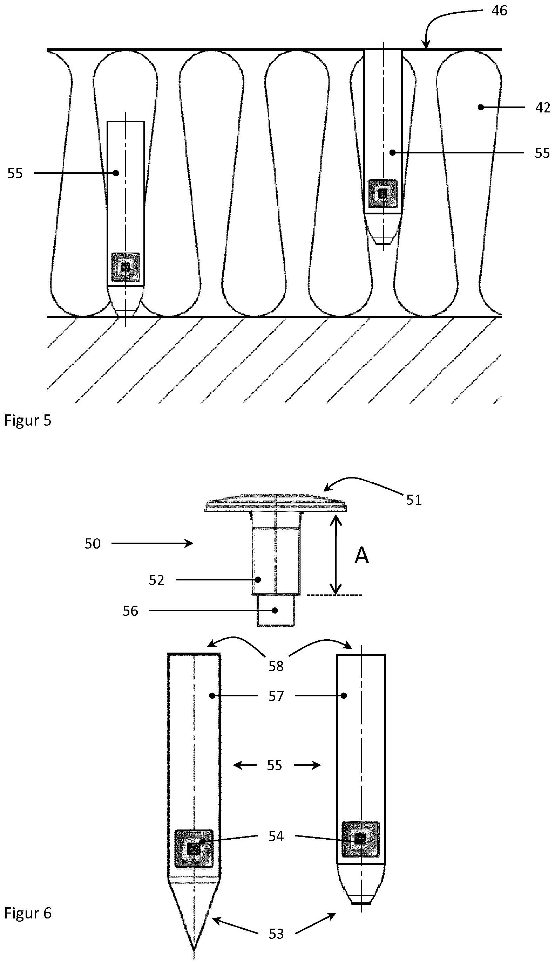

Figur 6 zeigt zwei Varianten der weiter oben beschriebenen bzw. in Figur 2 gezeigten "Einstecktülle" 30 mit unterschiedlichen Spitzenformen und -längen. Eine Besonderheit ist hierbei die Ausführung in zwei Teilen. Ein Oberteil 50 besteht vorrangig aus einem Kopf 51 und einem stabförmigen Körper 52. Dieser weist optional an seinem vom Kopf 51 abgewandten Ende einen Adapter 56 auf, welcher mit einem Gegenstück an einem Sensorträger 55 korrespondieren kann. Der Adapter kann ein Konus sein, ein Zylinder, ein Vierkant oder ein Vielkant. Das Gegenstück, eine Aufnahme 58, wird entsprechend so geformt, dass es kraft- oder formschlüssig verbunden werden kann. Die Platzierung von Adapter 56 und Aufnahme 58 kann natürlich auch umgekehrt werden.

Der Kopf 51 kann, wie gezeigt, scheibenförmig ausgeführt sein, könnte aber wiederum auch einen spezifisch geformten Angriff für ein (manuelles oder motorisiertes) Werkzeug bzw. eine Kupplung oder eine Schnittstelle zu einem Dreh- oder Schlagwerkzeug. Die Länge A in Figur 6 bestimmt in der gezeigten Ausführung die Setztiefe des Sensorträgers 55. Das Oberteil 50 insgesamt erfüllt somit im Wesentlichen die Aufgabe eines Setzwerkzeugs (Applikator) für den Sensorträger 55.

Der Sensorträger 55 selbst besteht aus einem stabförmigen Körper 57, auf bzw. in dem eine Sensoranordnung 54 angebracht ist. Zu den Möglichkeiten der Anbringung (Platzierung und Art) gilt das bereits oben Geschilderte sinngemäss auch hier. An einem Ende des Körpers 57 befindet sich eine Spitze 53, am anderen Ende die Aufnahme 58. Der Sensorträger 55 wird, mit der Spitze 53 voran, in die Isolationsschichten 42 des Dachaufbaus eingesteckt bzw. eingetrieben. Figur 5 zeigt hierbei die Einbausituation. Der Körper des Sensorträgers 55 kann dabei den Dimensionen und Materialien der oben für die Tülle Beschriebenen folgen, das ist jedoch nur bevorzugt, aber nicht zwingend. Der stabförmige Körper 57 kann einen runden, kreisrunden, (viel-)eckigen bzw. quadratischen Querschnitt aufweisen und kann als Hohlkörper oder Vollkörper hergestellt werden. Auch eine Mischung ist denkbar, z.B. mit einer massiven Spitze und einem hülsenförmigen Längskörper 57. Die oben beschriebene Aufnahme 58 ist bevorzugt, weil sie eine kontrollierte und planbare An- bzw. Einbringung des Sensorträgers erlaubt (Kontrolle von Winkel & Eindringtiefe). Wenn der Sensorträger jedoch, wie in Figur 5 rechts gezeigt, lediglich oberflächenbündig eingesteckt werden soll, so kann u.U. auf die Verwendung eines Werkezeugs verzichtet werden, dann würde die Anwendung des Oberteils entfallen und auch eine spezielle Aufnahme 58 wäre nicht notwendig.

In der einfachsten Ausführung umfasst somit ein Sensorträger 55 damit einen stabförmigen Voll- und/oder Hohl-Körper 57 mit einem runden, kreisrunden, (viel-)eckigen bzw. quadratischen Querschnitt, auf bzw. in dem eine Sensoranordnung 54 angebracht ist. Diese umfasst, wie oben beschrieben, einen RFID Transponder und einen Sensor. An einem Ende des stabförmigen Körpers befindet sich eine Spitze 53, am anderen Ende kann sich eine Aufnahme befinden, die form- und/oder kraftschlüssig mit einem Adapter 56 eines Oberteils 50 zusammenwirken kann. Das Oberteil 50 wirkt als Werkzeug zur Einbringung des Sensorträgers und hat einen Kopf 51, der eine Werkzeugaufnahme und/oder eine scheibenförmige Form aufweisen kann.

### BEZUGSZEICHENLISTE

- 10: Tülle
- 20: Tülle, Befestigungstülle
- 30, 48: Tülle, Einstecktülle
- 11: Kopf
- 12: Spitze
- 13: (rohrförmige) Hülse
- 14: (grossflächige) Unterlagscheibe
- 15: Konus-Verengung
- 16: axiale Öffnung
- 17: Bund, Anschlagbund
- 21: Kopf
- 22: Spitze
- 23: (rohrförmige) Hülse
- 24: Unterlagscheibe
- 25: Sensoranordnung
- 26: konusförmige Spitze
- 27: kegelförmige Spitze
- 28: Durchtrittsöffnung
- 29: Zentralachse
- 40: Befestigungselement
- 41: Befestiger
- 42: Isolationsschicht(en)
- 43: (fester) Unterbau
- 44: Tülle (ohne Durchtrittsöffnung 28)
- 45: Befestigungssystem für Gebäudehülle
- 46: Deckfolie
- 50: Oberteil
- 51: Kopf 51
- 52: (stabförmiger) Körper
- 53: Spitze
- 54: Sensoranordnung
- 55: Sensorträger
- 56: Adapter
- 57: (Stabförmiger) Körper
- 58: Aufnahme

## Patentansprüche

1. Eine Tülle (20, 30), bestehend im Wesentlichen aus einem Kopf (21), einer Spitze (22) und einer rohrförmigen Hülse (23) dazwischen, wobei der Kopf (21) als Unterlagscheibe (24) oder als Anschlagbund (17) ausgebildet sein kann, und die Spitze sich im Wesentlichen konus- (26) oder kegelförmig (27) vom Hülsendurchmesser auf einen kleineren Durchmesser verengt, wobei die Tülle (20, 30) eine Sensoranordnung (25) aufweist aus zumindest einem RFID Transponder mit Antenne und einen mit dem Transponder wirkverbundenen Sensor, **dadurch gekennzeichnet, dass** die Sensoranordnung (25) flächig auf der Aussenseite der Hülse (23) an der Spitze (22) angebracht ist, wodurch der Sensor in einer definierten Einbaulage und -tiefe in einer Schicht eines Dachaufbaus eingebracht werden kann.

2. Tülle (20) nach Anspruch 1, **dadurch gekennzeichnet, dass** die Spitze (22) eine zur Zentralachse (29) der Hülse (23) koaxiale Durchtrittsöffnung (28) aufweist.

3. Tülle (20) nach Anspruch 1-2, **dadurch gekennzeichnet, dass** die Tülle (20) Teil eines Befestigungselements (40) zum Befestigen von Isolationsschichten (42) auf einem festen Unterbau (43) ist und dieses Befestigungselement (40) neben der Tülle (20) einen Befestiger (41) umfasst.

4. Tülle (20) nach Anspruch 3, **dadurch gekennzeichnet, dass** der Befestiger (41) als Teil des Befestigungselements (40) mit der Tülle (20) eine Holzschraube, eine Blechschraube, eine Betonschraube, ein Bolzen oder ein Niet ist.

5. Tülle (20, 30) nach Anspruch 1 - 4, **dadurch gekennzeichnet, dass** die Sensoranordnung (25) lösbar mit der Tülle verbunden ist.

6. Tülle (20, 30) nach Anspruch 1-4, **dadurch gekennzeichnet, dass** die Sensoranordnung (25) unlösbar mit der Tülle verbunden ist, insbesondere aufgeklebt, angeheftet, angeschweisst, vergossen oder als Teil des Herstellvorgangs zumindest teilweise in die Tülle integriert wurde.

## Claims

1. A grommet (20, 30), substantially consisting of a head (21), a tip (22) and a tubular sleeve (23) therebetween, wherein the head (21) can be formed as a washer (24) or as a stop collar (17), and the tip narrows substantially conically (26) or in a tapered (27) manner from the sleeve diameter to a smaller diameter, wherein the grommet (20, 30) has a sensor arrangement (25) with at least one RFID transponder with antenna and a sensor operatively connected to the transponder, **characterized in that** the sensor arrangement (25) is attached flatly to the outside of the sleeve (23), to the washer h or to the tip (22), as a result of which the sensor can be introduced in a defined installation position and depth in a layer of a roof structure.

2. The grommet (20) according to clam 1, **characterized in that** the tip (22) has a through opening (28) coaxial with the central axis (29) of the sleeve (23) .

3. The grommet (20) according to claims 1 - 2, **characterized in that** the grommet (20) is part of a fastening element (40) for fastening insulation layers (42) to a fixed substructure (43) and this fastening element (40) comprises a fastener (41) in addition to the grommet (20).

4. The grommet (20) according to claim 3, **characterized in that** the fastener (41) as part of the fastening element (40) with the grommet (20) is a wood screw, a sheet metal screw, a concrete screw, a bolt or a rivet.

5. The grommet (20, 30) according to claims 1 - 4, **characterized in that** the sensor arrangement (25) is detachably connected to the grommet.

6. The grommet (20, 30) according to claims 1 - 4, **characterized in that** the sensor arrangement (25) is connected to the grommet in a non-detachable manner, in particular has been glued on, tacked on, welded on, molded on, or integrated at least partially into the grommet as part of the production process.

## Revendications

1. Douille (20, 30) composée pour l'essentiel d'une tête (21), d'une pointe (22) et d'un manchon de forme tubulaire (23) entre les deux, sachant que la tête (21) peut être constituée comme une rondelle d'assise (24) ou un collet de butée (17) et la pointe se rétrécit pour l'essentiel en forme de cône (26) ou de forme conique (27) du diamètre de manchon à un diamètre plus petit, sachant que la douille (20, 30) comporte un système de détection (25) d'au moins un transpondeur de radio-identification (RFID) avec antenne et un capteur relié de façon fonctionnelle au transpondeur, **caractérisée en ce que** le système de détection (25) est monté à plat sur le côté extérieur du manchon (23) sur la pointe (22), le capteur pouvant être incorporé de ce fait dans une position et profondeur de montage définie dans une couche d'une structure de toit.

2. Douille (20) selon la revendication 1, **caractérisée en ce que** la pointe (22) comporte une ouverture de passage (28) coaxiale à l'axe central (29) du manchon (23).

3. Douille (20) selon la revendication 1 - 2, **caractérisée en ce que** la douille (20) est une partie d'un élément de fixation (40) pour fixer des couches d'isolation (42) sur une infrastructure fixe (43) et cet élément de fixation (40) comprend un organe de fixation (41) en plus de la douille (20) .

4. Douille (20) selon la revendication 3, **caractérisée en ce que** l'organe de fixation (41) est une vis à bois, une vis à tôle, une vis à béton, un boulon ou un rivet en tant que partie de l'élément de fixation (40) à la douille (20).

5. Douille (20, 30) selon la revendication 1 - 4, **caractérisée en ce que** le système de détection (25) est relié de façon amovible à la douille.

6. Douille (20, 30) selon la revendication 1 - 4, **caractérisée en ce que** le système de détection (25) est relié non amovible à la douille, en particulier collé, agrafé, soudé, moulé ou a été au moins en partie intégré dans la douille en tant que partie de l'opération de fabrication.
